# FASCICULE DE BREVET EUROPEEN

(11) **EP 0 549 782 B1**
(45) Date de publication et mention de la délivrance du brevet: **11.09.1996**
(21) Numéro de dépôt: 92916487.9
(22) Date de dépôt: 16.07.1992
(51) Int. Cl.: C12M 3/06

(54) **BIOREACTEUR ET DISPOSITIF POUR LA CULTURE DE CELLULES ANIMALES**
BIOREAKTOR UND VORRICHTUNG ZUR KULTIVIERUNG VON TIERISCHEN ZELLEN
BIOREACTOR AND DEVICE FOR THE CULTURE OF ANIMAL CELLS

(30) Priorité: 19.07.1991 FR 9109159
(43) Date de publication de la demande: 07.07.1993
(73) Titulaire: COMMISSARIAT A L'ENERGIE ATOMIQUE, 75015 Paris (FR)
(72) Inventeur: BESNAINOU, Bernard, F-13100 Aix-en-Provence (FR); PHILIPPE, Alain, F-84120 Pertuis (FR); LESSART, Pierre, F-04130 Volx (FR)
(74) Mandataire: Dubois-Chabert, Guy
(86) Numéro de dépôt international: FR9200691
(87) Numéro de publication internationale: WO9302179

(56) Documents cités:
- WO-A-90/06990
- DE-A- 3 539 609
- US-A- 4 178 209
- US-A- 5 002 890
- PATENT ABSTRACTS OF JAPAN vol. 9, no. 254 (C-308)(1977) 11 Octobre 1985

## Description

La présente invention concerne un bioréacteur et un dispositif pour la culture en continu et à l'échelle industrielle de cellules animales (qu'elles soient en suspension, microsupportées ou microencapsulées). Ce dispositif permet la production de toutes les cellules animales notamment les plus fragiles, et des métabolites produits par ces cellules, comme par exemple les anticorps monoclonaux.

On connaît déjà divers bioréacteurs pouvant être utilisés en culture de cellules libres (non adhérentes).

Certains de ces bioréacteurs sont basés sur le confinement des cellules entre deux types de membranes filtrantes différentes, les unes servant à apporter certains substrats aux cellules et les autres servant à l'extraction de certains produits. Toutefois, ces bioréacteurs ne possèdent aucun dispositif de régulation et de contrôle intermédiaire de la perte de charge et présentent donc des fractions de membranes mal utilisées. Enfin, ces bioréacteurs ont souvent des capacités de transfert de matière assez faible.

Le document FR-A-2 640 638 décrit un bioréacteur comportant une chambre de culture cellulaire longitudinale disposée horizontalement. Cette chambre est traversée de part en part longitudinalement par deux ensembles de tubes minéraux poreux, reliés entre eux à l'une de leurs extrémités par une conduite de liaison et reliés par leur autre extrémité à un réservoir de milieu nourricier. Ces tubes poreux présentent des membranes filtrantes microporeuses autorisant le passage des molécules du milieu nourricier vers la chambre de culture cellulaire, et de ladite chambre vers les tubes poreux, mais bloquant le passage des cellules cultivées dans la chambre.

Selon les caractéristiques de ce bioréacteur, la conduite de liaison entre ces deux ensembles de tubes est équipée d'une vanne permettant d'ajuster et de contrôler la perte de charge dans cette conduite et d'obtenir ainsi un transfert important de matière sensiblement constant sur toute la longueur des tubes minéraux poreux. En outre, afin de limiter le colmatage des membranes déposées sur la surface des deux ensembles de tubes poreux, des moyens sont prévus pour inverser le sens de circulation du milieu nourricier à l'intérieur des deux ensembles de tubes. Enfin, l'homogénéité du milieu de culture se trouvant dans la chambre de culture cellulaire est obtenue par des moyens permettant une rotation alternative de 180° du bioréacteur autour d'un axe longitudinal parallèle aux deux tubes poreux.

Toutefois on constate que ce type de bioréacteur présente un certain nombre d'inconvénients.

En effet, malgré la rotation du bioréacteur de 180°, certains types de cellules se trouvant à l'intérieur de la chambre de culture peuvent avoir tendance à sédimenter dans le fond de ladite chambre.

Enfin, la régulation du pH et de la pression partielle en oxygène se fait dans un réacteur auxiliaire et non directement dans le bioréacteur.

On connaît également d'après l'abrégé de la demande de brevet JP-A-60 110 282 un outil d'agitation destiné à être placé dans une chambre de culture cellulaire et à permettre l'introduction d'oxygène dans le milieu de culture. Cet outil ne permet toutefois pas d'introduire le milieu nourricier dans la culture.

L'invention a justement pour but de remédier à ces inconvénients.

A cet effet, l'invention concerne un bioréacteur pour la culture de cellules animales comportant :
- une chambre de culture cellulaire délimitée par une virole cylindrique interne et deux parois en regard, cette chambre renfermant à la fois lesdites cellules animales et le milieu de culture,
- deux enceintes situées à l'extérieur de ladite chambre de culture, une enceinte d'entrée et une enceinte de sortie, ces deux enceintes étant reliées l'une à l'autre.

Selon les caractéristiques de l'invention, ce bioréacteur comprend au moins deux tourniquets disposés sensiblement verticalement à l'intérieur de ladite chambre de culture, un tourniquet d'entrée comportant au moins un tube poreux minéral ou organique reliant ladite enceinte d'entrée à l'une des extrémités d'une conduite de liaison entre les deux tourniquets, un tourniquet de sortie comportant au moins un autre tube poreux minéral ou organique reliant l'autre extrémité de ladite conduite de liaison à l'enceinte de sortie, ces deux tourniquets étant munis de moyens pour les faire tourner en sens inverse autour d'un axe longitudinal. Chacun de ces tubes poreux présente sur ses faces intérieure et extérieure une membrane filtrante microporeuse imperméable aux cellules présentes dans la chambre de culture cellulaire, mais permettant le passage des molécules du milieu nourricier vers la chambre de culture cellulaire et inversement le prélèvement des métabolites produits dans la chambre de culture cellulaire.

Ainsi, dans ce bioréacteur selon l'invention et contrairement à ce qui existait pour l'art antérieur (FR-A-2 640 638), la chambre de culture cellulaire reste immobile et ce sont les tourniquets portant les tubes poreux qui se déplacent à l'intérieur de cette chambre. De préférence, chaque tourniquet présente trois tubes poreux et deux tourniquets voisins tournent en sens inverse.

Il en résulte une remise en suspension permanente des cellules beaucoup plus efficace que ce qui existait dans l'art antérieur. De plus, l'agitation assurée par la rotation des tourniquets est beaucoup plus douce que ce qui pouvait être obtenu par d'autres moyens d'agitation, comme par exemple des hélices ; et les faibles forces de cisaillement permettent la culture, dans de bonnes conditions, de cellules animales même très fragiles.

En conséquence, il en résulte une meilleure homogénéité de la culture cellulaire ainsi qu'une augmentation du transfert au sein du milieu de la chambre de culture, grâce au renouvellement par diffusion et par brassage du liquide environnant ces tubes.

Selon une autre caractéristique de l'invention, la chambre de culture cellulaire est remplie seulement partiellement de milieu de culture et de cellules animales, de façon à définir dans sa partie supérieure un volume rempli de gaz, maintenus sous pression. Le brassage assuré par les tourniquets améliore encore la diffusion de ces gaz à l'intérieur du milieu de culture.

On prévoit en outre, une canalisation permettant l'introduction d'azote, d'oxygène et éventuellement de dioxyde de carbone. De façon avantageuse, cette canalisation débouche dans la partie supérieure de la chambre de culture.

Ceci permet ainsi une régulation aisée et très précise de la pression partielle en oxygène dissous, dans le milieu de culture, et éventuellement, dans des milieux de culture particuliers, ainsi qu'une régulation du pH par apport du dioxyde de carbone.

Selon une autre caractéristique de l'invention, la chambre de culture cellulaire est munie dans sa partie basse de deux bossages pour l'introduction de diverses sondes de mesures, comme par exemple des sondes mesurant la pression partielle en oxygène dissous ou en gaz carbonique, ou encore des sondes mesurant le pH ou la température.

L'invention a également pour objet un dispositif pour la culture en continu de cellules animales comprenant un réacteur de contrôle et de préparation du milieu nourricier, connecté à un bioréacteur tel que défini précédemment, et des moyens pour extraire en continu ou séquentiellement les substances produites par les cellules cultivées (métabolites), ces moyens étant connectés audit réacteur de contrôle.

D'autres caractéristiques et avantages de l'invention ressortiront mieux de la description qui va suivre, donnée à titre illustratif et non limitatif, en faisant référence aux dessins annexés, dans lesquels :
- la figure 1 représente un schéma d'ensemble d'un dispositif de culture de cellules animales conforme à l'invention ;
- la figure 2 représente de façon plus détaillée et en coupe longitudinale le bioréacteur du dispositif de la figure 1 ; et
- les figures 3 et 4 représentent respectivement des vues en coupe de deux modes de réalisation des tourniquets présents à l'intérieur du bioréacteur.

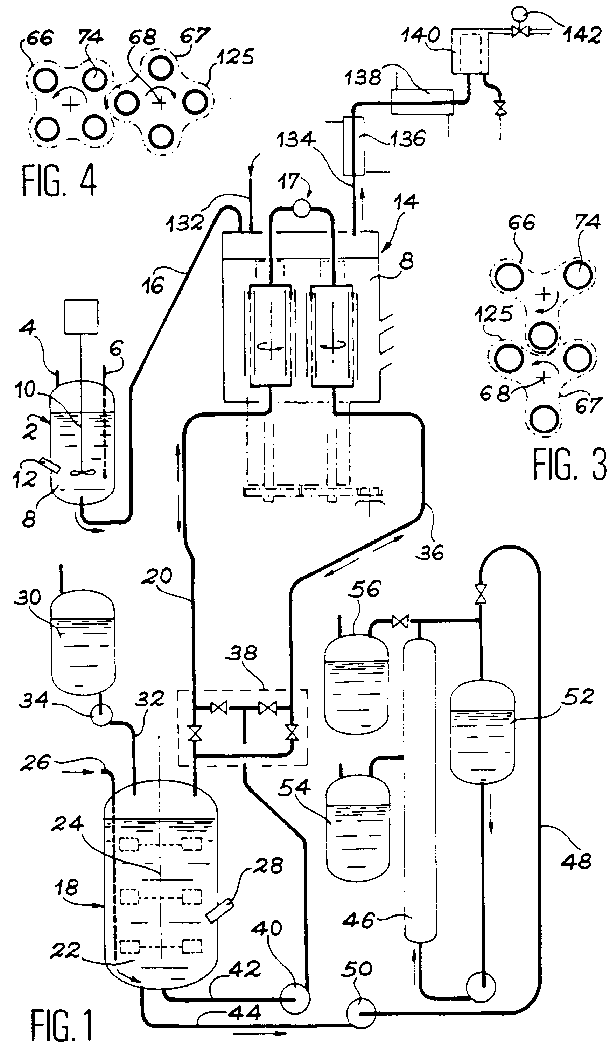

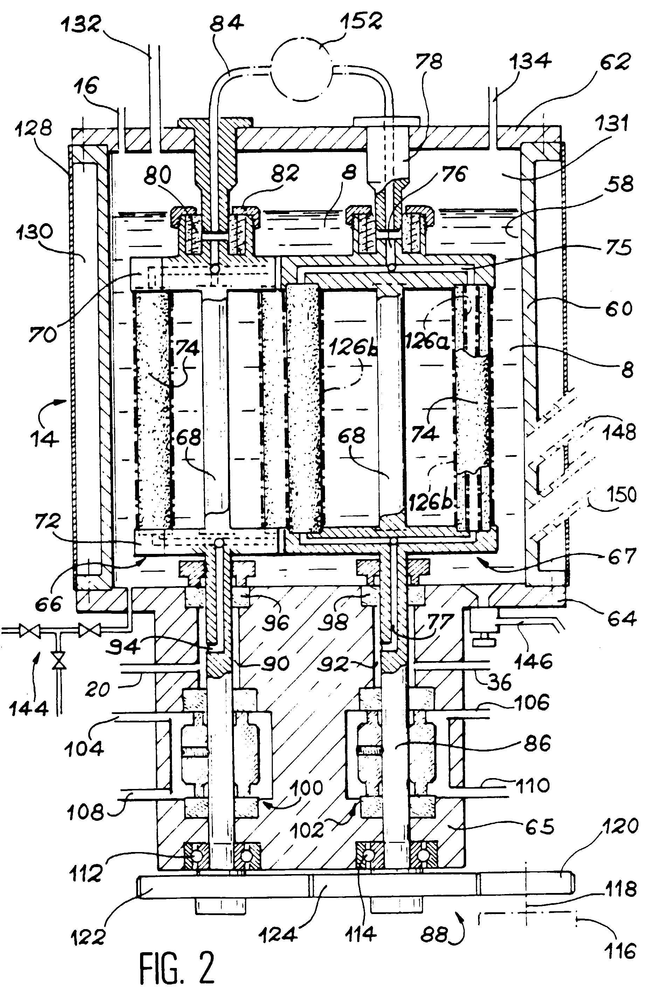

En faisant référence à la figure 1, le dispositif pour la culture de cellules animales selon l'invention comprend une cuve 2 destinée à la préculture des cellules animales, équipée d'une alimentation 4 en gaz et d'une alimentation 6 en solution nutritive. Le mélange gazeux et la solution nutritive sont nécessaires au développement des cellules animales contenues dans le milieu de culture 8. Un agitateur 10 assure une agitation lente du milieu de culture 8. Des capteurs 12 pour le contrôle de ce milieu et en particulier du pH, de la pression d'oxygène et de la pression en dioxyde de carbone sont prévus. La cuve 2 et son équipement sont utilisés comme "innoculum" du bioréacteur 14.

La cuve 2 est reliée, via une conduite 16 au bioréacteur 14 dans lequel s'effectue la culture à proprement parler des cellules animales pour la production de métabolites par ces cellules. Le bioréacteur comporte de préférence un plan de symétrie longitudinal 17.

Le bioréacteur 14 est connecté à ur réacteur auxiliaire 18 destiné à la préparation du milieu nourricier et au contrôle de ce dernier. Cette connexion est effectuée par une conduite 20. Le milieu nourricier 22 est nécessaire à la vie des cellules animales contenues dans le milieu de culture 8 se trouvant à l'intérieur du bioréacteur 14.

Une première régulation du pH, de la pression en oxygène, de la pression en dioxyde de carbone, ainsi que des divers éléments constitutifs du milieu nourricier 22 tels que la glutamine, le glucose et les acides aminés est effectuée dans ce réacteur auxiliaire 18. Un système 24 permet son agitation. L'absence de cellules dans le réacteur 18 permet l'utilisation d'une agitation brutale du milieu nourricier 22 (les contraintes de cisaillement ne sont pas limitatives) et d'un fort débit de mélange gazeux, car il y a possibilité d'utiliser un système antimousse mécanique. Le mélange gazeux est introduit via une conduite d'amenée 26 dans le réacteur 18.

Ce réacteur 18, comme tous les autres canalisations et réservoirs du dispositif sont stérilisables in situ à la vapeur.

Le réacteur auxiliaire 18 est également équipé d'un dispositif 28 permettant des prélèvements stériles dans le milieu nourricier 22 et assurant un prélèvement continu ou séquentiel d'échantillons injectés automatiquement dans des analyseurs reliés à un système informatique qui contrôle ce milieu nourricier 22. Ce système permet l'injection à la demande, de glucose, de glutamine ou d'acides aminés stockés stérilement dans des réservoirs 30 (un seul est représenté en figure 1) connectés au réacteur 18 via des conduites 32 équipées de pompes 34.

Le système de stérilisation de l'ensemble du dispositif ainsi que le contrôle du milieu de culture et du milieu nourricier, effectués de façon automatique, sont bien connus de l'homme de l'art et ne seront pas décrits plus en détail.

Le bioréacteur 14 est en outre équipé d'une conduite 36 de sortie du milieu nourricier 22, connectée au réacteur 18.

Un dispositif de vannes 38 permet d'inverser périodiquement le sens de circulation du milieu nourricier 22 à l'intérieur des tubes poreux du bioréacteur 14, afin d'éviter l'encrassement ou le colmatage des membranes déposées sur ceux-ci.

Une pompe 40 montée sur une conduite rigide 42 de sortie du milieu nourricier 22 du réacteur auxiliaire 18 et connectée au dispositif de vanne 38, assure la mise en circulation de ce milieu nourricier.

Les métabolites produits par les cellules dans le bioréacteur 14 sont extraits du réacteur annexe 18 par une conduite 44 connectée à un système 46 d'ultrafiltration assurant la séparation entre les métabolites et les autres produits du milieu nourricier liquide 22. L'acheminement des ces métabolites dans le système d'ultrafiltration 46 est assuré via une conduite d'amenée 48 connectée à la conduite 44 et équipée d'une pompe 50 et d'un réservoir tampon 52. Des réservoirs 54 et 56 assurent la récolte des produits obtenus par l'ultrafiltration. Le réservoir 56 permet la récupération des métabolites de poids moléculaire élevé. Le réservoir 54 permet la récupération des milieux usés et ceux-ci peuvent être régénérés par divers traitements comme par exemple par pervaporation de façon à éliminer certains métabolites toxiques produits par les cellules.

Le bioréacteur 14 selon l'invention est représenté en détail sur la figure 2. Il comprend une chambre de culture cellulaire 58 délimitée par une virole cylindrique interne 60 s'étendant sensiblement verticalement. Cette virole 60 présente à chacune de ses deux extrémités deux parois en regard, respectivement une paroi supérieure 62 et une paroi inférieure 64 formant une partie de la base 65 du bioréacteur.

Deux tourniquets 66, 67 sont disposés sensiblement verticalement à l'intérieur de la chambre de culture 58. Chaque tourniquet 66, 67 comprend un axe central longitudinal 68, aux deux extrémités duquel sont fixés deux flasques, respectivement un flasque supérieur 70 et un flasque inférieur 72. Des tubes poreux 74 réalisés par exemple en carbone poreux sont fixés entre les deux flasques 70 et 72, parallèlement à l'axe 68 (deux seulement sont représentés en figure 2). Les deux flasques 70 et 72 sont creusés intérieurement de façon à former plusieurs canalisations 75 reliant chaque extrémité d'un tube poreux 74 à une conduite commune, verticale, (référencée 76, en partie supérieure du tourniquet et 77 en partie inférieure). Ces conduites 76, 77 s'étendant de part et d'autre de l'axe central 68.

En outre, la paroi supérieure 62 est traversée par deux portions de tube 78 dirigées verticalement vers l'intérieur de la chambre de culture 58 et situées en regard des conduites 76. Chaque flasque supérieur 70 est surmonté de joints d'étanchéité tournants 80, réalisés par exemple en téflon, maintenus sur ce flasque par une bague de serrage 82. Ces joints en téflon 80 permettent d'assurer la rotation de chaque tourniquet 66 autour de la portion de tube 78. Les deux portions de tube 78 sont reliées dans leur partie supérieure, par une conduite de liaison 84 se trouvant à l'extérieur du bioréacteur.

Les canalisations 77 situées dans la partie inférieure de chaque tourniquet sont ménagées à l'intérieur d'un axe 86 vertical entraîné en rotation par des moyens 88 qui seront décrits ultérieurement.

Le bioréacteur 14 selon l'invention présente également deux enceintes annulaires 90, 92 prévues respectivement sous le tourniquet d'entrée 66 et sous le tourniquet de sortie 67. Ces enceintes annulaires 90 et 92 sont prévues autour des axes verticaux 86. Les canalisations 77 débouchent à l'intérieur desdites enceintes 90 et 92 par un orifice 94. Ces deux enceintes annulaires 90 et 92 sont limitées respectivement par une garniture mécanique supérieure 96, 98 et par une double garniture mécanique inférieure 100, 102. Les deux doubles garnitures mécaniques inférieures 100, 102 sont lubrifiées par des condensats stériles introduits sous forme de vapeur en 104, (respectivement 106), et évacués sous forme de condensats en 108 (respectivement 110). On prévoit encore autour de chaque axe 86 et sous les doubles garnitures mécaniques 100 et 102, des roulements à billes respectivement 112 et 114 facilitant la rotation des axes 86 et donc des deux tourniquets 66 et 67.

Ces garnitures mécaniques pourraient bien évidemment être remplacées par tout autre système de joints d'étanchéité tournants.

Les moyens moteurs 88 comprennent un moteur 116 à proprement parler, muni d'un axe de sortie 118 surmonté d'un pignon 120. En outre, l'axe de rotation 86 du tourniquet 66 présente une roue dentée 122 engrenant avec une seconde roue dentée 124 prévue respectivement sur l'axe de rotation 86 du tourniquet 67. La roue d'engrenage 124 engrène également avec le pignon 120. Les deux tourniquets 66 et 67 sont ainsi entraînés en rotation et tournent en sens inverse, ce qui améliore le brassage. Toutefois, on pourrait supprimer les roues dentées 122, 124 et faire tourner les deux tourniquets dans un sens quelconque avec des moteurs indépendants l'un de l'autre.

Les tourniquets 66, 67 supportant les tubes poreux 74 sont animés d'un mouvement de rotation lent qui permet le maintien en suspension des cellules et assure une bonne homogénéité de la culture de cellules. A titre d'exemple, les tourniquets tournent à une vitesse de 5 à 100 tr/mn, de préférence 10 à 40 tr/mn.

De plus, lorsque les tourniquets sont entraînés par des roues dentées, les flasques 70, 72 ne sont pas circulaires, mais présentent un contour irrégulier à trois ou quatres lobes 125, par exemple, comme illustré aux figures 3 et 4. Les tubes poreux 74 sont fixés au niveau de ces lobes. Cette structure en lobes permet de disposer les deux tourniquets 66 et 67 de façon qu'ils soient légèrement imbriqués l'un dans l'autre, mais sans qu'il y ait de frottements. En d'autres termes, chaque lobe 125 du tourniquet 66 passe entre deux lobes 125 voisins du tourniquet 67 mais sans qu'il y ait de contact entre ces lobes. Cette disposition des deux tourniquets 66 et 67 assure un meilleur brassage de la culture lors de leur rotation.

Sur chaque tube poreux 74, minéral ou organique, est déposée respectivement une membrane filtrante microporeuse, minérale ou organique, interne 126a et externe 126b. Lorsque cette membrane est minérale, elle peut être réalisée par exemple avec des oxydes tels que Al₂O₃, TiO₂, ZrO₂ ou SiO₂, du carbone, des carbures tels que SiC ou des nitrures. Lorsque cette membrane est organique, elle peut être réalisée par exemple avec des polysulfones. Ces membranes filtrantes 126a, 126b permettent en particulier le prélèvement des métabolites produits par les cellules cultivées dans la chambre de culture cellulaire et inversement le passage des molécules du milieu nourricier vers cette chambre de culture 58. Par contre, ces membranes 126a, 126b sont imperméables aux cellules. A titre d'exemple, ces membranes peuvent présenter un diamètre de pores de l'ordre de 0,09 micromètre.

La virole cylindrique 60 interne est entourée d'une seconde virole cylindrique 128 externe afin de former un espace annulaire 130 entre les deux viroles, dans lequel on fait circuler un liquide assurant la régulation en température du milieu de culture 8. La chambre de culture cellulaire 58 est remplie seulement partiellement de milieu de culture 8 et de cellules animales de façon à définir dans sa partie supérieure un volume 131 ou "ciel" rempli de gaz maintenus sous pression par des moyens qui seront décrits ultérieurement.

Le bioréacteur 14 selon l'invention comprend également dans sa partie supérieure une canalisation 132 traversant la paroi supérieure 62 et permettant l'introduction de gaz tels l'azote, l'oxygène et le dioxyde de carbone.

Le bioréacteur comprend également dans sa partie supérieure une canalisation 134 traversant la paroi supérieure 62 et permettant la sortie des gaz humides. Comme illustré en figure 1, cette canalisation 134 traverse successivement un réfrigérant 136, un réchauffeur 138, un filtre stérilisable à la vapeur 140 et un manomètre détendeur 142 formant un moyen pour maintenir sous pression les gaz du volume 131. Le réfrigérant 136 permet de perdre moins de liquide lors de l'étape de condensation. Le réchauffeur 138 permet d'éviter la condensation sur le filtre 140, et le filtre 140 permet de stériliser les gaz qui seront ensuite relâchés dans l'atmosphère.

Un appareil antimousse statique, non représenté, tel que celui développé par DOWNICK HUNTER FILTER sous la marque TURBOSEP, peut être intercalé entre le réfigérant 136 et le réchauffeur 138.

Enfin, comme illustré en figure 2, le bioréacteur 14 selon l'invention comprend un ensemble de vannes de prise d'échantillons 144, une vanne de purge 146, un bossage 148 pour l'implantation d'une sonde de mesure du pH du milieu de culture et un bossage 150 pour l'implantation d'une sonde de mesure de la pression partielle en oxygène du milieu de culture.

Le fonctionnement du dispositif va maintenant être décrit.

Le milieu nourricier 22 riche en éléments nécessaires à la culture des cellules et provenant du réacteur auxiliaire 18 arrive par exemple dans l'enceinte 90, par la canalisation 20. Ce liquide nourricier 22 pénètre dans la canalisation 77 de l'axe 86 et traverse en parallèle les tubes poreux 74 du tourniquet d'entrée 66 et alimente le milieu de culture 8 des cellules, en milieu nourricier frais. Ensuite, le milieu nourricier 22 passe à travers la canalisation de liaison 84 et redescend dans les tubes poreux 74 du tourniquet de sortie 67, jusqu'à déboucher dans l'enceinte 92 et à repartir vers le réacteur auxiliaire 18 par la canalisation 36. Il se produit au niveau des tubes poreux 74 du tourniquet 67 le transfert du milieu nourricier partiellement appauvri et des métabolites formés par les cellules.

Le passage du milieu nourricier à travers la barrière poreuse 126 est assuré grâce à une surpression du milieu nourricier par rapport au milieu de culture. A cet effet, le bioréacteur 14 présente au niveau de sa conduite externe de liaison 84 mettant en communication les deux tourniquets, une vanne de réglage 152 permettant de contrôler et d'ajuster la perte de charge dans ladite conduite de liaison 84 et d'augmenter la pression dans les tubes poreux 74 du tourniquet d'entrée 66. Le liquide appauvri en éléments nutritifs retourne au réacteur auxiliaire 18 en vue de se recharger en éléments nutritifs.

Afin de limiter le colmatage des membranes déposées sur les faces des tubes poreux 74 et obtenir ainsi des caractéristiques de transfert sensiblement constantes pendant toute la durée de la culture, un ensemble de vannes 38 est prévu pour inverser le sens de passage du milieu nourricier à l'intérieur desdits tubes poreux 74. Dans ces conditions, les sens de circulation sont inversés, et le tourniquet d'entrée devient le tourniquet de sortie et réciproquement.

Dans l'exemple de réalisation représenté ici, chaque tourniquet comprend de préférence trois tubes poreux 74. Toutefois, de façon pratique, on utilise en fait un ensemble de tubes, par exemple de quatre, six, douze ou dix-huit tubes, voire même davantage, disposés régulièrement sur chaque tourniquet à l'intérieur du bioréacteur. Toutefois, le bioréacteur de l'invention est d'autant plus performant qu'il comporte un plus grand nombre de tubes poreux 74 destinés à l'alimentation en milieu nourricier. On pourrait également prévoir plus de deux tourniquets.

Afin d'assurer la connexion alternée des tourniquets d'entrée et de sortie, chaque tourniquet comporte généralement le même nombre de tubes poreux 74, et il y a de préférence autant de tourniquets d'entrée que de tourniquets de sortie.

La forme et le mode de fonctionnement du dispositif conforme à l'invention ont été sélectionnés pour qu'une production continue de métabolites, sur une longue période, soit possible même avec un milieu de culture non exempt de protéines. La limitation du colmatage des barrières poreuses (membrane ou tube) est obtenue grâce au choix judicieux de la membrane microporeuse décrite ci-dessus, mais également en limitant les pressions de transfert entre les tubes, et en inversant séquentiellement le sens de passage du liquide nourricier dans les tubes poreux 74.

## Revendications

1. Bioréacteur pour la culture de cellules animales comportant :
- une chambre de culture cellulaire (58) délimitée par une virole cylindrique interne (60) et deux parois (62, 64) en regard, cette chambre (58) renfermant à la fois lesdites cellules animales et le milieu de culture,
- deux enceintes (90, 92) situées à l'extérieur de ladite chambre de culture (58), une enceinte d'entrée et une enceinte de sortie, ces deux enceintes (90, 92) étant reliées l'une à l'autre,
caractérisé en ce qu'il comprend au moins deux tourniquets (66, 67) disposés à l'intérieur de ladite chambre de culture (58), un tourniquet d'entrée comportant au moins un tube poreux (74) minéral ou organique reliant ladite enceinte d'entrée (90) à l'une des extrémités d'une conduite de liaison (84) entre les deux tourniquets, un tourniquet de sortie comportant au moins un autre tube poreux (74) minéral ou organique reliant l'autre extrémité de la conduite de liaison (84) à l'enceinte de sortie (92), ces deux tourniquets (66, 67) étant munis de moyens (88) pour les faire tourner en sens inverse autour d'un axe longitudinal (68, 86) et en ce que chacun de ces tubes poreux (74) présente sur ses faces intérieure et extérieure une membrane filtrante microporeuse (126a, 126b), imperméable aux cellules présentes dans la chambre de culture cellulaire (58) mais permettant le passage des molécules du milieu nourricier (22) vers la chambre de culture cellulaire (58) et inversement le prélèvement des métabolites produits dans la chambre de culture cellulaire (58).

2. Bioréacteur selon la revendication 1, caractérisé en ce qu'il comprend une canalisation (132) permettant l'introduction d'azote, d'oxygène et/ou de dioxyde de carbone dans la chambre de culture (58).

3. Bioréacteur selon la revendication 1 ou 2, caractérisé en ce que la chambre de culture cellulaire (58) est remplie seulement partiellement de milieu de culture (8) et de cellules animales, de façon à définir dans sa partie supérieure un volume (131) rempli de gaz maintenus sous pression par des moyens (142).

4. Bioréacteur selon les revendications 2 et 3, caractérisé en ce que la canalisation (132) débouche dans la partie supérieure du bioréacteur à l'intérieur au volume (131).

5. Bioréacteur selon la revendication 1, caractérisé en ce que les tourniquets (66, 67) et les tubes poreux (74) sont disposés sensiblement verticalement à l'intérieur de la chambre de culture cellulaire (58).

6. Bioréacteur selon la revendication 1 ou 5, caractérisé en ce que les tubes poreux (74) de chaque tourniquet (66, 67) sont disposés entre deux flasques (70, 72) et en ce que chaque flasque (70, 72) est creusé de façon à former plusieurs canalisations internes (75) reliant chacune des extrémités d'un tube poreux (74) à une conduite commune (76, 77).

7. Bioréacteur selon la revendication 6, caractérisé en ce que les flasques (70, 72) de chaque tourniquet (66, 67) présentent un contour irrégulier de façon à former des lobes (125) et en ce que les tubes poreux (74) sont fixés au niveau de ces lobes.

8. Bioréacteur selon la revendication 7, caractérisé en ce que deux tourniquets (66, 67) voisins sont disposés de façon que leurs lobes (125) respectifs soient imbriqués l'un dans l'autre.

9. Bioréacteur selon l'une quelconque des revendications précédentes, caractérisé en ce que la chambre de culture cellulaire (58) est munie dans sa partie basse d'un bossage (148) pour l'introduction d'une sonde de détermination du pH du milieu de culture (8).

10. Bioréacteur selon l'une quelconque des revendications précédentes, caractérisé en ce que la chambre de culture cellulaire (58) est munie dans sa partie basse d'un bossage (150) pour l'introduction d'une sonde de mesure de la pression en oxygène dissous du milieu de culture (8).

11. Bioréacteur selon la revendication 1, caractérisé en ce que la conduite de liaison (84) entre les deux tourniquets (66, 67) est équipée d'une vanne de réglage (152) pour contrôler et ajuster la pert de charge dans ladite conduite (84).

12. Bioréacteur selon la revendication 1, caractérisé en ce que la membrane filtrante microporeuse (126a, 126b) est une membrane minérale.

13. Bioréacteur selon la revendication 1, caractérisé en ce que la membrane filtrante microporeuse (126a, 126b) est une membrane organique.

14. Bioréacteur selon l'une quelconque des revendications 1 à 13, caractérisé en ce qu'une virole externe (128) entourant la virole interne (60) est prévue pour former un espace annulaire (130) destiné à la circulation d'un fluide de régulation en température du milieu de culture (8).

15. Bioréacteur selon l'une quelconque des revendications 1 à 14, caractérisé en ce que des moyens (38) sont prévus pour inverser périodiquement le sens de circulation du milieu nourricier (22) dans les tubes poreux (74) des deux tourniquets (66, 67).

16. Bioréacteur selon la revendication 1, caractérisé en ce que les moyens de rotation (88) comprennent un moteur (116) entraînant en rotation des roues dentées (122, 124) prévues sur les tourniquets (66, 67).

17. Bioréacteur selon la revendication 1, caractérisé en ce que les enceintes (90, 92) sont limitées respectivement par des joints tournants (96, 98) et par des joints tournants (100, 102) lubrifiés par des condensats stériles.

18. Dispositif pour la culture en continu de cellules animales comprenant un réacteur auxiliaire (18) de préparation du milieu nourricier (22), connecté à un bioréacteur selon l'une quelconque des revendications précédentes et des moyens (44, 46, 50, 52) pour extraire en continu ou séquentiellement les métabolites produits par les cellules cultivées, ces moyens étant connectés audit réacteur auxiliaire (18).

19. Dispositif selon la revendication 18, caractérisé en ce qu'il comprend une canalisation (134) permettant l'évacuation des gaz humides du bioréacteur, cette canalisation (134) traversant successivement un réfrigérant (136), un réchauffeur (138), un filtre (140) et un manomètre détendeur (142).

## Patentansprüche

1. Bioreaktor zur Kultivierung von tierischen Zellen, umfassend:
- eine Kultivierungskammer (58), begrenzt durch einen zylindrischen Innenmantel und zwei gegenüberstehende Wände (62, 64), wobei diese Kammer (58) zugleich besagte tierische Zellen einschließt und den Nährboden,
- zwei Behälter (90, 92), außerhalb der Kultivierungskammer (58) befindlich, einen Eingangsbehälter und einen Ausgangsbehälter, wobei diese beiden Behälter (90, 92) miteinander verbunden sind,
**dadurch gekennzeichnet,**
daß er wenigstens zwei Drehkreuze (66, 67) umfaßt, angeordnet im Innern der Kultivierungskammer (58), wobei ein Eingangsdrehkreuz wenigstens ein mineralisches oder organisches poröses Rohr (74) umfaßt, das den besagten Eingangsbehälter (90) verbindet mit einem der Enden einer Verbindungsleitung (84) zwischen den beiden Drehkreuzen, ein Ausgangsdrehkreuz, wenigstens ein weiteres mineralisches oder organisches poröses Rohr (74) umfassend, das das andere Ende der Verbindungsleitung (84) mit dem Ausgangsbehälter (92) verbindet, diese beiden Drehkreuze (66, 67) mit Einrichtungen (88) versehen sind, um sie in Gegenrichtung drehen zu können um eine Längsachse (68, 86), und dadurch, daß jedes dieser porösen Rohre (74) auf seinen Innen- und Außenflächen eine mikroporöse Filtriermembran (126a, 126b) aufweist, undurchdringlich für die in der Kultivierungskammer (58) vorhandenen Zellen, jedoch durchlässig für die Moleküle des Nährmittels (22) in Richtung der Kultivierungskammer (58) und umgekehrt die Entnahme der in der Kultivierungskammer (58) erzeugten Metaboliten.

2. Bioreaktor nach Anspruch 1, dadurch gekennzeichnet, daß er eine Leitung (132) zum Einleiten von Stickstoff, Sauerstoff und/oder Kohlenstoffdioxid in die Kultivierungskammer (58) umfaßt.

3. Bioreaktor nach Anspruch 1 oder 2, dadurch gekennzeichnet, daß die Kultivierungskammer (58) nur teilweise mit Nährboden (8) und tierischen Zellen gefüllt ist, um in ihrem oberen Teil ein Volumen (131) zu definieren, gefüllt mit Gas, das durch Einrichtungen (142) unter Druck gehalten wird.

4. Bioreaktor nach einem der Ansprüche 2 und 3, dadurch gekennzeichnet, daß die Leitung (132) in dem oberen Teil des Bioreaktors mündet, innerhalb des Volumens (131).

5. Bioreaktor nach Anspruch 1, dadurch gekennzeichnet, daß die Drehkreuze (66, 67) und die porösen Rohre (74) im Innern der Kultivierungskammer (58) im wesentlichen senkrecht angeordnet sind.

6. Bioreaktor nach Anspruch 1 oder 5, dadurch gekennzeichnet, daß die porösen Rohre (74) von jedem Drehkreuz (66, 67) zwischen zwei Flanschen (70, 72) angeordnet sind, und daß jeder Flansch (70, 72) derart durchbohrt ist, daß er mehrere interne Leitungen (75) bildet, die jedes der Enden eines porösen Rohrs (74) mit einer gemeinsamen Leitung (76, 77) verbinden.

7. Bioreaktor nach Anspruch 6, dadurch gekennzeichnet, daß die Flansche (70, 72) von jedem Drehkreuz (66, 67) eine unregelmäßige Form aufweisen indem sie Lappen (125) bilden, und dadurch, daß die porösen Rohre (74) in diesen Lappen befestigt sind.

8. Bioreaktor nach Anspruch 7, dadurch gekennzeichnet, daß die beiden benachbarten Drehkreuze (66, 67) so angeordnet sind, daß ihre Lappen (125) ineinandergreifen.

9. Bioreaktor nach einem der vorangehenden Ansprüche, dadurch gekennzeichnet, daß die Kultivierungskammer (58) in ihrem unteren Teil mit einer Öffnung (148) zur Einführung einer Sonde zum Bestimmen des ph-Werts des Nährbodens (8) versehen ist.

10. Bioreaktor nach einem der vorangehenden Ansprüche, dadurch gekennzeichnet, daß die Kultivierungskammer (58) in ihrem unteren Teil mit einer Öffnung (150) zur Einführung einer Sonde zum Messen des Drucks des in dem Nährboden (8) gelösten Sauerstoffs versehen ist.

11. Bioreaktor nach Anspruch 1, dadurch gekennzeichnet, daß die Verbindungsleitung (84) zwischen den beiden Drehkreuzen (66, 67) mit einem Regulierungsventil (152) ausgestattet ist, um den Druckverlust in dieser Leitung (84) zu kontrollieren und zu regulieren.

12. Bioreaktor nach Anspruch 1, dadurch gekennzeichnet, daß die mikroporöse Filtriermembran (126a, 126b) eine mineralische Membran ist.

13. Bioreaktor nach Anspruch 1, dadurch gekennzeichnet, daß die mikroporöse Filtriermembran (126a, 126b) eine organische Membran ist.

14. Bioreaktor nach einem der Ansprüche 1 bis 13, dadurch gekennzeichnet, daß ein Außenmantel (128), der den Innenmantel (60) umgibt, vorgesehen ist, um einen ringförmigen Raum (130) zu bilden, bestimmt für das Zirkulieren eines Temperaturregelungsfluids des Nährbodens (8).

15. Bioreaktor nach einem der Ansprüche 1 bis 14, dadurch gekennzeichnet, daß Einrichtungen zum periodischen Umkehren der Zirkulationsrichtung des Nährmittels (22) in den porösen Rohren (74) der beiden Drehkreuze (66, 67) vorgesehen sind.

16. Bioreaktor nach Anspruch 1, dadurch gekennzeichnet, daß die Dreheinrichtungen (88) einen Motor (116) umfassen, der Zahnräder (122, 124) antreibt, die an den Drehkreuzen (66, 67) vorgesehen sind.

17. Bioreaktor nach Anspruch 1, dadurch gekennzeichnet, daß die Behälter (90, 92) begrenzt sind durch Drehdichtungen bzw. - verbindungen (96, 98) und durch Drehverbindungen (100, 102), geschmiert durch sterile Kondensate.

18. Vorrichtung zur kontinuierlichen Kultivierung von tierischen Zellen, umfassend einen Hilfsreaktor (18) zur Bereitung des Nährmittels (22), verbunden mit einem Bioreaktor nach einem der vorangehenden Ansprüche, und Einrichtungen (44, 46, 50, 52), um kontinuierlich oder sequentiell die durch die kultivierten Zellen erzeugten Metaboliten zu entziehen, wobei diese Einrichtungen mit besagtem Hilfsreaktor (18) verbunden sind.

19. Vorrichtung nach Anspruch 18, dadurch gekennzeichnet, daß sie eine Leitung (134) umfaßt, die das Ausströmen von feuchten Gasen des Bioreaktors ermöglicht, wobei diese Leitung (134) nacheinander eine Kühleinrichtung (136), eine Wiederaufwärmeinrichtung (138), ein Filter (140) und ein Druckminderventil mit Manometer (142) durchquert.

## Claims

1. Bioreactor for the culture of animal cells comprising a cell culture chamber (58) defined by an inner cylindrical ferrule (60) and two facing walls (62, 64), said chamber (58) containing both said animal cells and the culture medium and two enclosures (90, 92) positioned outside said culture chamber (58), namely an inlet enclosure and an outlet enclosure, said two enclosures (90, 92) being interconnected, characterized in that it comprises at least two tourniquets (66, 67) positioned within the said culture chamber (58), an inlet tourniquet having at least one mineral or organic porous tube (74) connecting said inlet enclosure (90) to one of the ends of a connecting duct (84) between the two tourniquets, an outlet tourniquet having at least one other organic or mineral porous tube (74) connecting the other end of the connecting duct (84) to the outlet enclosure (92), said two tourniquets (66, 67) being provided with means (88) for making them rotate in opposite directions about a longitudinal shaft (68, 86) and in that each of said porous tubes (74) has on its inner and outer faces a microporous filter membrane (126a, 126b), which is impermeable to the cells present in the cell culture chamber (58), but which permits the passage of the molecules fron the nutrient medium (22) to the cell culture chamber (58) and conversely the sampling of the metabolites produced in the cell culture chamber (58).

2. Bioreactor according to claim 1, characterized in that it comprises a duct (132) permitting the introduction of nitrogen, oxygen and/or carbon dioxide into the culture chamber (58).

3. Bioreactor according to claim 1 or 2, characterized in that the cell culture chamber (58) is only partly filled with culture medium (8) and animal cells, so as to define in its upper part a volume (131) filled with gases maintained under pressure by the means (142).

4. Bioreactor according to claims 2 and 3, characterized in that the duct (132) issues into the upper part of the bioreactor within the volume (131).

5. Bioreactor according to claim 1, characterized in that the tourniquets (66, 67) and the porous tubes (64) are positioned substantially vertically within the cell culture chamber (58).

6. Bioreactor according to claim 1 or 5, characterized in that the porous tubes (74) of each tourniquet (66, 67) are positioned between two flanges (70, 72) and in that each flange (70, 72) is hollowed out so as to form several inner ducts (75), each connecting the ends of a porous tube (74) to a common pipe (76, 77).

7. Bioreactor according to claim 6, characterized in that the flanges (70, 72) of each tourniquet (66, 67) have an irregular contour so as to form lobes (125) and in that the porous tubes (74) are fixed level with said lobes.

8. Bioreactor according to claim 7, characterized in that two adjacent tourniquets (66, 67) are positioned in such a way that their respective lobes (125) are nested in one another.

9. Bioreactor according to any one of the preceding claims, characterized in that the cell culture chamber (58) is provided in its lower part with a boss (148) for the introduction of a probe for determining the pH-value of the culture medium (8).

10. Bioreactor according to any one of the preceding claims, characterized in that the cell culture chamber (58) is provided in its lower part with a boss (150) for the introduction of a probe for measuring the dissolved oxygen pressure of the culture medium (8).

11. Bioreactor according to claim 1, characterized in that the connecting duct (84) between the two tourniquets (66, 67) is equipped with a regulating valve (152) for checking and adjusting the pressure drop in said duct (84).

12. Bioreactor according to claim 1, characterized in that the microporous filter membrane (126a, 126b) is a mineral membrane.

13. Bioreactor according to claim 1, characterized in that the microporous filter membrane (126a, 126b) is an organic membrane.

14. Bioreactor according to any one of the claims 1 to 13, characterized in that an outer ferrule (128) surrounding the inner ferrule (60) is provided for forming an annular space (130) for the circulation of a fluid for the temperature regulation of the culture medium (8).

15. Bioreactor according to any one of the claims 1 to 14, characterized in that means (38) are provided for periodically reversing the flow direction of the nutrient medium (22) in the porous tubes (74) of the two tourniquets (66, 67).

16. Bioreactor according to claim 1, characterized in that the rotation means (88) comprise a motor (116) rotating cog wheels (122, 124) located on the tourniquets (66, 67).

17. Bioreactor according to claim , characterized in that the enclosures (90, 92) are respectively defined by rotary packings (96, 98) and by rotary packings (100, 102) lubricated by sterile condensates.

18. Apparatus for the continuous culture of animal cells incorporating an auxiliary reactor (18) for the preparation of the nutrient medium (22), connected to a bioreactor according to any one of the preceding claims, and means (44, 46, 50, 52) for the continuous or sequential extraction of the metabolites produced by the cultured cells, said means being connected to said auxiliary reactor (18).

19. Apparatus according to claim 18, characterized in that it comprises a pipe (134) permitting the discharge of moist gases from the bioreactor, said pipe (134) successively traversing a cooler (136), a heater (138), a filter (140) and a pressure reduction gauge (142).
